# EUROPEAN PATENT SPECIFICATION

(11) **EP 1 380 318 B1**
(45) Date of publication and mention of the grant of the patent: **04.07.2012**
(21) Application number: 03006294.7
(22) Date of filing: 24.03.2003
(51) Int. Cl.: A61N 1/368

(54) **A cardiac stimulating device**
Gerät zur Herzstimulierung
Appareil pour la stimulation cardiaque

(30) Priority: 12.07.2002 SE 0202214
(43) Date of publication of application: 14.01.2004
(73) Proprietor: St. Jude Medical AB, 175 84 Järfälla (SE)
(72) Inventor: Holmström, Nils, 17557 Järfälla (SE); Obel, Martin, 18253 Danderyd (SE); Björling, Anders, 17553 Järfälla (SE); Hedberg, Sven-Erik, 19632 Kungsängen (SE); Uhrenius, Asa, 1 tr, 11330 Stockholm (SE); Budgifvars, Göran, 16351 Spanga (SE)

(56) References cited:
- WO-A-01/10499
- WO-A-02/43584
- US-A- 5 174 289
- US-A1- 2001 012 953
- US-A1- 2002 077 559
- CAZEAU S ET AL: "FOUR CHAMBER PACING IN DILATED CARDIOMYOPATHY" PACE, XX, XX, vol. 17, no. 11, 1 November 1994 (1994-11-01), pages 1974-1979, XP000618630 ISSN: 0147-8389
- MEHDIRAD A A ET AL: "INTERRELATIONS BETWEEN QRS MORPHOLOGY, DURATION, AND HV INTERVAL CHANGES FOLLOWING RIGHT BUNDLE BRANCH RADIOFREQUENCY CATHETER ABLATION" PACE - PACING AND CLINICAL ELECTROPHYSIOLOGY, FUTURA PUBLISHING COMPANY, INC, US, vol. 21, no. 6, 1 June 1998 (1998-06-01), pages 1180-1188, XP000772433 ISSN: 0147-8389

## Description

The present invention relates to an implantable cardiac stimulating device according to the preamble of the attached independent claim. More precisely, the invention concerns such a stimulating device comprising a housing, a control circuit enclosed in the housing, said control circuit being adapted to be connected to a first electrode to be positioned to stimulate a first ventricle of the heart. The control circuit is also adapted to be connected to a second electrode to be positioned to stimulate a second ventricle of the heart. The control circuit also comprises means for delivering stimulating pulses. Furthermore, the device comprises means for sensing a far field ECG similar to a surface ECG.

### 2. Description of prior art

Most pacers are arranged to stimulate the right ventricle of the heart but it is also known to stimulate the left ventricle. In particular for treatment of congestive heart failure it is known to stimulate the left ventricle, or both ventricles, in order to optimize the hemodynamic performance of the heart. The improvement in hemodynamic performance that can be obtained is due to improved synchronization between the right and left ventricles.

US-A-5 174 289 describes a method to sequentially pace the right ventricle at one or several locations to obtain the shortest possible QRS duration combined with an improved ventricular motion.

US-A-6 233 082 describes a system and method for providing multiple chamber pacing of a patient's heart and in particular, pacing programmed for treatment of various forms of heart failure. Impedance sensing in the heart provides timing of mechanical contraction, and the pacemaker controls pacing to maintain bi-ventricular mechanical synchronization adjusted for maximum cardiac output.

US-A-5 728 140 describes a method and an apparatus for pacing the left ventricle of the heart. The pacing electrode is positioned within the interventricular septum proximate to stimulate or sense the different chambers of the heart.

Also the article "A Method for Permanent Transvenous Left Ventricular Pacing" by Blanc et al, PACE Vol 21, 1998, pp. 2021-2024, describes a method for positioning leads for left ventricular pacing.

The article "Four Chamber Pacing in Dilated Cardiomyopaty" by S Cazeau et al, PACE Vol 17, 1994, pp 1974-1979, describes favourable clinical results with four chamber pacing. In one example QRS duration was found to decrease from 200 to 160 ms with four chamber pacing.

US-A 4 928 688 describes a method and an apparatus for treating patients suffering from congestive heart failure by stimulating both the ventricles. The document discusses the problem involved when the left and right ventricles contract in asynchrony. In order to effect substantially simultaneous contraction of both ventricles, the document suggests means for separately processing sensed cardiac signals from each of the left and right ventricles. If ventricular contractions are not sensed in both ventricles within a period of coincidence defined by a time delay, the pacing pulse will be emitted at the end of this delay, but only to the ventricle for which a QRS-complex has not been sensed. The time delay is suggested to be in the order of 5-10 ms.

International publication WO 01/10499 A1 describes a biventricular pacer comprising means for measuring the evoked response signal from the right and left ventricle respectively. The time interval dT between the stimulation pulses to the right and left ventricles is adapted so that the evoked response signal from the two ventricles occur simultaneously.

WO 02/43584 describes a system and method for determining mean pulmonary arterial pressure (MPAP) without the use of a sensor located within the pulmonary artery. The system comprises a sensor located in a ventricle of a heart to measure pressure, a circuit to measure electrocardiogram (EGM) signals, and a processing circuit to derive the mean pulmonary arterial pressure (MPAP) from the pressure and EGM signals. The measurements of the intercardiac pressure signal and the EGM signal are utilized in order to derive a number of parameters and an estimate of the MPAP value is determined based on the derived parameters. The estimated MPAP value may be used to monitor and control cardiac resynchronization therapy. For example, the V-V timing interval associated with pulses delivered in the left and right ventricles may be adjusted based on the MPAP estimates.

### SUMMARY OF THE INVENTION

The invention is defined in claim 1. The purpose of pacing the left and right ventricles with separate leads is to improve the synchronization of the mechanical contraction of the two ventricles. The synchronization of the ventricles may be important for patients with severe congestive heart failure. These patients are often waiting for a heart transplant and optimal hemodynamic conditions during the time before the transplant is important for the outcome. A problem in this context is that synchronous pacing will not necessarily provide the best possible synchronization of the actual contraction of the ventricles. Impedance measurements have previously been used to determine mechanical synchronization of the ventricles. The inventors have found that the use of impedance measurements is related to some problems in interpreting the impedance signal to determine optimal mechanical synchronization. The invention is based on the fact that optimal mechanical syncronization is present when the two ventricles are stimulated in a sequence that produces a surface ECG QRS with the shortest duration. Surface ECG QRS duration has been used to determine effectiveness of biventricular pacing but it has never been used for automatic adjustment of the time interval between pacing pulses to the two ventricles. The effect on QRS duration by biventricular pacing is described in the above referenced article by S Cazeau et al. It is thus an object of the present invention to provide an implantable cardiac stimulating device by means of which the synchronization of the ventricles is improved.

The object of the invention is obtained by an implantable cardiac stimulating device as defined in the opening paragraph of the description having the characterizing features of claim 1.

According to the invention, the stimulating pulses to the ventricles are delivered at such times that a farfield ECG measured at measuring points outside of the heart but inside the body gives the narrowest achievable QRS on a farfield ECG.

According to a further embodiment of the invention the stimulating pulses to the ventricles are delivered at such times that the morphology of a farfield ECG signal measured at measuring points outside the heart fulfills certain predetermined morphological criteria.
According to a further embodiment of the invention the implantable medical device comprises implantable electrodes located at a small distance from the implantation site but outside of the heart. The farfield ECG signal obtained from these electrodes is used for determination of the the optimal interval dT between the stimulation pulses to the right and left ventricles respectively. These electrodes may be implemented as ring electrodes located on the electrode leads to be placed inside the heart.

### BRIEF DESCRIPTION OF THE DRAWINGS

Fig 1 shows schematically an implanted biventricular pacemaker system.
Fig 2 shows schematically an implanted biventricular pacemaker system with far-field ECG monitoring through a short farfield ECG monitoring electrode.
Fig 3 shows schematically an implanted biventricular pacemaker system with farfield ECG monitoring through an electrode dot located on the implanted pulse generator.
Fig 4 shows schematically an implanted biventricular pacemaker system with far-field ECG monitoring through ring electrodes placed on the endocardial pacing electrodes.
Fig 5 shows a schematic drawing of the control circuit in the implantable medical device.
Fig 6 shows examples of surface ECG:s for right ventricular pacing, left ventricular pacing and biventricular pacing respectively.
Fig 7 shows an example of a ECG obtained with right atrial stimulation and stimulation of both ventricles.

### DESCRIPTION OF PREFERRED EMBODIMENTS

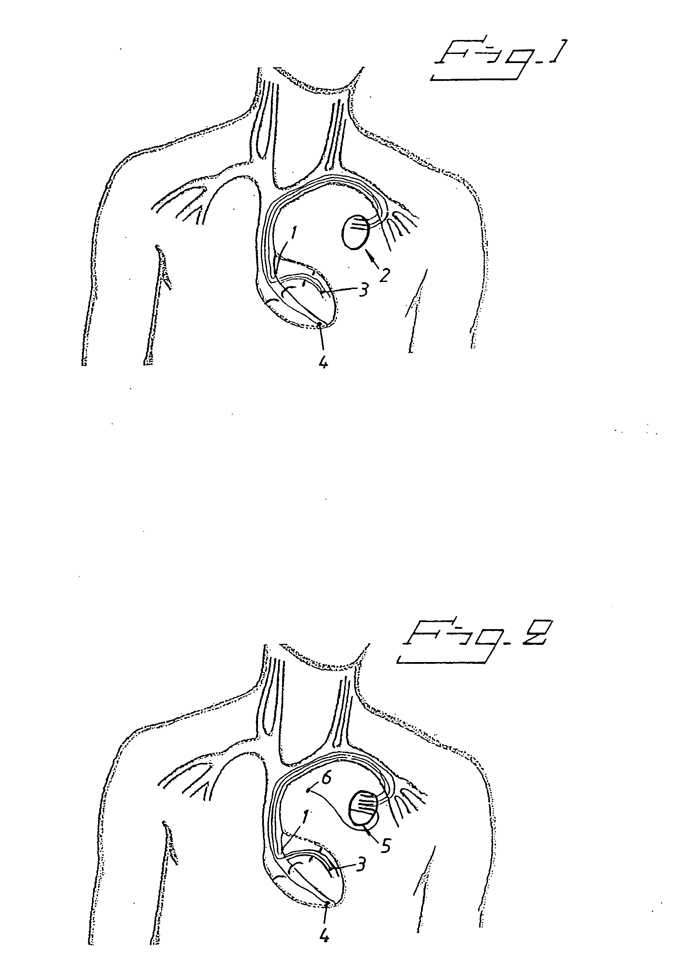

Fig 1 shows a biventricular pacemaker as implanted. In this example an atrial electrode 1 is provided for atrial sensing/pacing by the implanted pulse generator 2. Further a left ventricular electrode 3 is provided for left ventricular sensing/pacing. The heart lead electrode 3 is implanted via the coronary sinus vein to a preferred position epicardially on the left ventricle. For right ventricular sensing/pacing a conventional right ventricular electrode 4 is provided. In some embodiments a fourth left atrial heart lead electrode is provided for left atrial sensing/pacing.

Fig 2 shows a biventricular pacemaker according to the invention as implanted. A short nonendocardial electrode 6 is provided for sensing a far-field ECG from a position outside of the heart. In the figure it is assumed that the far-field ECG is sensed between the pulse generator 5 encapsulation and the short nonendocardial electrode 6. There may be several short nonenedcardial electrodes to provide different farfield ECG sensing configurations. In the absence of atrial signals pacing pulses are delivered to the atrial electrode 1. Following an atrial sensed/paced event an AV interval is initiated. At the end of the AV interval pacing pulses are delivered to the right ventricular electrode 4 and to the left ventricular electrode 3. Following the delivery of the ventricular pacing pulses a far-field QRS is sensed via the short nonendocardial electrode 6 and the pulse generator 5 encapsulation. In an iterative search process the interval dT between the right ventricular pacing pulse and the left ventricular pacing pulse is varied until the evoked far-field QRS with the shortest duration is found. Alternatively the interval between the right ventricular pacing pulse and the left ventricular pacing pulse is varied until the evoked QRS-T fulfils predetermined morphological criteria such as the presence of a visible S-T segment. Other morphological criteria may be the surface under the positive and negative deflection of the QRS or the order in which negative and positive deflections occur or the duration of the most significant deflection in the far-field ECG signal. A far-field ECG configuration similar to configuration V1 in a surface ECG is an advantageous configuration even though any configuration can be used with appropriate adaption of the analysis of the farfield ECG signal.

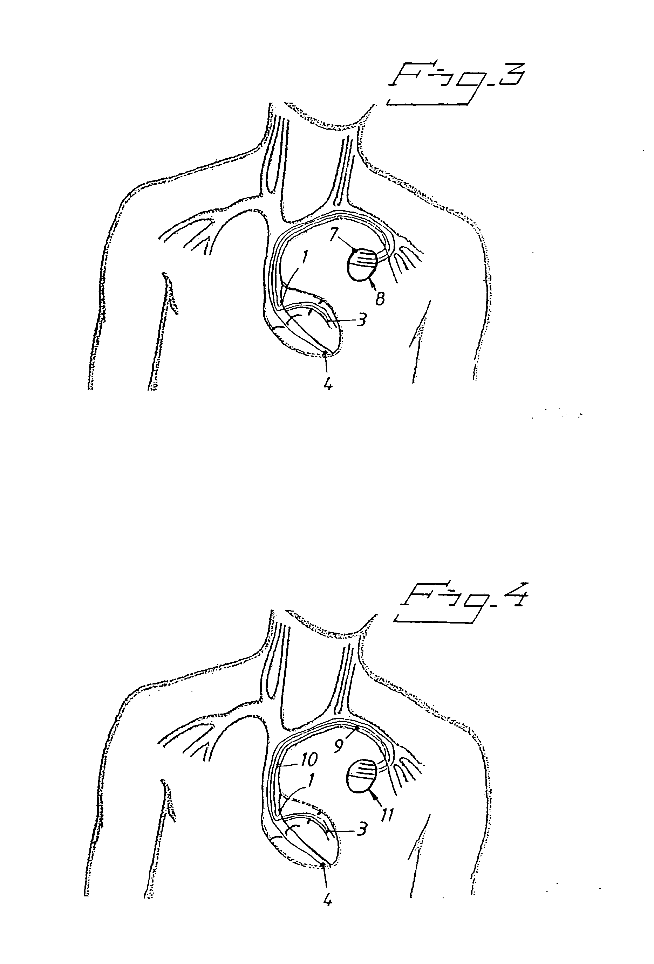

Fig 3 shows a biventricular pacemaker according to the invention. A farfield ECG sensing electrode dot 7 is placed on the connector top and the farfield ECG is sensed between the dot 7 and the encapsulation of the pulse generator 8. In order to obtain several configurations of the farfield ECG several dots can be placed on the encapsulation and on the connector top. Regarding the function of the pulse generator the description for fig 2 is applicable also for this fig.

Fig 4 shows a biventricular pacemaker according to the invention. One or more of the endocardial heart electrode leads 1,3 is provided with ring electrodes 9,10 to be placed outside the heart for sensing a farfield ECG. A particularly advantageous method to connect the ring electrodes 9,10 the the control unit of the pulse generator may be to use the future electrode connector standard IS-4 which provides 4 individual contacts for each IS-4 connector. The farfield surface ECG can be obtained between the pulse generator encapsulation and any of the ring electrodes 9,10 or between the ring electrodes 9,10. This will provide several configurations for farfield ECG sensing.

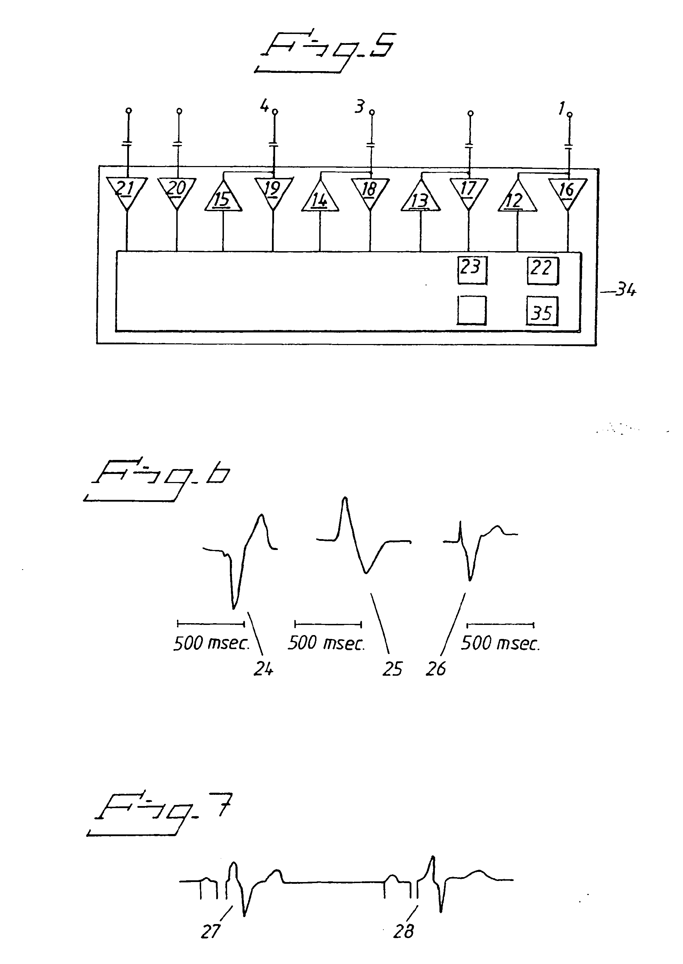

Fig 5 shows a block diagram of a control circuit 34 used in the pulse generator 2, 5, 11. The control circuit comprises means 12, 13, 14, 15 for delivering stimulation pulses to electrodes 1, 3, 4. The control circuit is adapted for a general configuration where right and left atria as well as right and left ventricles are paced. However in many cases the system comprises one atrial electrode and two ventricular electrodes. The control circuit further comprises sense amplifiers 16, 17, 18, 19 for sensing atrial and ventricular activity respectively. Amplifiers 20 and 21 are used for sensing a farfield ECG signal from locations outside of the heart. The control circuit also comprises means 22 arranged to enable the delivery of stimulating pulses to the first 3 and the second 4 ventricular electrodes within the same cycle of the heart such that there may be a time interval dT between the stimulating pulses. The time interval dT may be varied. Furthermore the control circuit comprises means 23 for analyzing the farfield ECG signal obtained via amplifiers 20 and 21. The analysis may be QRS duration, QRS morphology, duration of most significant deflection, or ST segment visibility. In operation when biventricular pacing is executed the evoked QRS is analyzed for each biventricular stimulaton. Further the control circuit comprises means 35 for varying the interval dT in response to the farfield WCG analysis performed by means 23. In a search process the interval dT is varied and when the evoked QRS fulfills certain criteria then the synchronization between the ventricles is considered to be optimal. The criteria may be minimum QRS duration or other morphological criteria.

Fig 6 shows samples of surface ECG (V1) obtained during pacing. Complex 24 is obtained during right ventricular pacing, complex 25 is obtained during left ventricular pacing, while complex 26 is obtained during biventricular simultaneous right and left ventricular stimulation. As can be seen in surface ECG configuration V1 the order of deflections are opposite when comparing LV and RV pacing. Other ECG configurations can also be employed for analyzing evoked QRS parameters that indicate synchronization between the ventricles. When biventricular pacing is applied the QRS is significantly shortened, the QRS has a unique morphology, there is a visible S-T segment. A farfield ECG obtained from a location under the skin is similar to a surface ECG obtained from a similar location on the surface of the skin. The surface ECG shows a greater variability due to polarization and contact problems with the ECG electrodes. This problem is not present with electrodes located under the skin for farfield sensing of the ECG signal. Thus a farfield ECG signal shows a better stability compared to a normal surface ECG signal.

Fig 7 shows a schematic ECG indicating the function of a biventricular pacemaker utilizing the invention. In the first complex 27 an atrial stimulation pulse is indicated followed by by biventricular stimulation pulses which are followed by the evoked QRS. The evoked QRS is obtained from surface ECG lead V1 but a similar signal can be obtained through electrodes implanted under the skin at a location close to the corresponding surface ECG lead. The evoked QRS is analyzed by means 23 in the control circuit. As a result of the analysis of the evoked QRS the interval dT between the ventricular pacing pulses is shortened in the next complex 28. The shortening results in a shorter evoked QRS and a a longer S-T segment which indicates a better synchronization between the right and left ventricles. In this manner the pacemaker can make a search to find the optimal interval dT between the ventricular stimulation pulses. The sequence between the stimulation pulses depends on the electrode location and on the patient's myocardial condition. Thus in some patients the right ventricular stimulation pulse is followed by the left ventricular stimulation pulse and in some patient's the sequence is reversed. The described search process will however find the optimal sequence and interval dT.

## Claims

1. An implantable cardiac stimulating device comprising:
a housing (5, 8, 11),
a control circuit (34) enclosed in said housing, said control circuit (34) being connectable to a first electrode (3) to be positioned to stimulate a first ventricle of a heart,
said control circuit also being connectable to a second electrode (4) to be positioned to stimulate a second ventricle of said heart,
said control circuit (34) also being connectable to at least one electrode (6, 7, 9, 10) located at a distance from the heart for sensing a far-field ECG,
said control circuit comprising means (20, 21) arranged for sensing at least one farfield ECG signal after stimulating said first and second ventricles respectively,
said control circuit (34) comprising means (14, 15) for delivering stimulation pulses to said first (3) and second (4) electrodes in order to stimulate said first and second ventricles respectively,
wherein the control circuit (34) comprises means (22) arranged to enable the delivery of the stimulating pulses to said first (3) and second (4) electrodes within the same cycle of the heart such that there is a time interval (dT) between them, wherein the control circuit (34) is arranged such that said time interval (dT) is variable,
**characterized in that** the control circuit (34) comprises means (23) adapted to analyze at least one parameter in said farfield ECG, said parameter being a measure of synchronization between said first and second ventricles,
said control circuit (34) comprising means (35) to vary the time interval (dT) between the stimulating pulses in response to the farfield ECG analysis performed by said analysis means (23) until said synchronization between said first and second ventricles is optimized.

2. An implantable cardiac stimulating device according to claim 1 **characterized in that** said parameter in the farfield ECG is the QRS duration.

3. An implantable cardiac stimulating device according to claim 1 **characterized in that** said parameter in the farfield ECG is the QRS morphology.

4. An implantable cardiac stimulating device according to claim 1-3 **characterized in that** at least one short nonendocardial electrode (6) is used for sensing said farfield ECG.

5. An implantable cardiac stimulating device according to claim 1 **characterized in that** at least one electrode dot (7) is located on the implantable heart stimulator encapsulation for sensing said far-field ECG.

6. An implantable cardiac stimulating device according to claim 1-3 **characterized in that** at least one ring electrode (9, 10) located on one endocardial electrode is utilized for sensing said farfield ECG.

## Patentansprüche

1. Implantierbares Gerät zur Herzstimulierung, umfassend:
ein Gehäuse (5, 8, 11),
eine Steuerschaltung (34), die in dem Gehäuse eingeschlossen ist, wobei die Steuerschaltung (34) mit einer ersten Elektrode (3) verbunden werden kann, die platziert werden soll, um eine erste Kammer eines Herzes zu stimulieren,
wobei die Steuerschaltung auch mit einer zweiten Elektrode (4) verbunden werden kann, die platziert werden soll, um eine zweite Kammer des Herzes zu stimulieren,
wobei die Steuerschaltung (34) auch mit mindestens einer Elektrode (6, 7, 9, 10) verbunden werden kann, die sich in einem Abstand zum Herz befindet, um ein Fernfeld-EKG zu erfassen,
wobei die Steuerschaltung Mittel (20, 21) umfasst, die angeordnet sind, um nach der Stimulation der ersten beziehungsweise zweiten Kammer mindestens ein Fernfeld-EKG-Signal zu erfassen,
wobei die Steuerschaltung (34) Mittel (14, 15) zum Abgeben von Stimulationsimpulsen an die erste (3) und zweite (4) Elektrode umfasst, um die erste beziehungsweise zweite Kammer zu stimulieren,
wobei die Steuerschaltung (34) Mittel (22) umfasst, die angeordnet sind, um die Abgabe der Stimulationsimpulse an die erste (3) und zweite (4) Elektrode im selben Zyklus des Herzes zu ermöglichen, sodass ein Zeitintervall (dT) zwischen ihnen liegt, wobei die Steuerschaltung (34) so angeordnet ist, dass das Zeitintervall (dT) veränderlich ist,
**dadurch gekennzeichnet, dass** die Steuerschaltung (34) Mittel (23) umfasst, die angepasst sind, mindestens einen Parameter in dem Fernfeld-EKG zu analysieren, wobei der Parameter ein Maß der Synchronisation zwischen der ersten und zweiten Kammer ist,
wobei die Steuerschaltung (34) Mittel (35) umfasst, um das Zeitintervall (dT) zwischen den Stimulationsimpulsen als Reaktion auf die Fernfeld-EKG-Analyse, die von den Analysemitteln (23) durchgeführt wird, zu verändern, bis die Synchronisation zwischen der ersten und zweiten Kammer optimiert ist.

2. Implantierbares Gerät zur Herzstimulierung nach Anspruch 1, **dadurch gekennzeichnet, dass** der Parameter im Fernfeld-EKG die QRS-Dauer ist.

3. Implantierbares Gerät zur Herzstimulierung nach Anspruch 1, **dadurch gekennzeichnet, dass** der Parameter im Fernfeld-EKG die QRS-Morphologie ist.

4. Implantierbares Gerät zur Herzstimulierung nach Anspruch 1 bis 3, **dadurch gekennzeichnet, dass** mindestens eine kurze nicht-endokardiale Elektrode (6) zum Erfassen des Fernfeld-EKGs verwendet wird.

5. Implantierbares Gerät zur Herzstimulierung nach Anspruch 1, **dadurch gekennzeichnet, dass** sich an der Verkapselung des implantierbaren Herzstimulators mindestens ein Elektrodenpunkt (7) befindet, um das Fernfeld-EKG zu erfassen.

6. Implantierbares Gerät zur Herzstimulierung nach Anspruch 1 bis 3, **dadurch gekennzeichnet, dass** mindestens eine Ringelektrode (9, 10), die sich an einer endokardialen Elektrode befindet, zum Erfassen des Fernfeld-EKGs verwendet wird.

## Revendications

1. Appareil implantable pour la stimulation cardiaque comprenant :
un boîtier (5, 8, 11),
un circuit de commande (34) enfermé dans ledit boîtier, ledit circuit de commande (34) pouvant être connecté à une première électrode (3) destinée à être positionnée pour stimuler un premier ventricule d'un coeur,
ledit circuit de commande pouvant également être connecté à une seconde électrode (4) destinée à être positionnée pour stimuler un second ventricule dudit coeur,
ledit circuit de commande (34) pouvant également être connecté à au moins une électrode (6, 7, 9, 10) située à distance du coeur pour capter un ECG en champ lointain,
ledit circuit de commande comprenant des moyens (20, 21) agencés pour capter au moins un signal d'ECG en champ lointain après la stimulation respectivement desdits premier et second ventricules,
ledit circuit de commande (34) comprenant des moyens (14, 15) pour fournir des impulsions de stimulation auxdites première (3) et seconde (4) électrodes afin de stimuler respectivement lesdits premier et second ventricules,
dans lequel le circuit de commande (34) comprend des moyens (22) agencés pour permettre la fourniture des impulsions de stimulation auxdites première (3) et seconde (4) électrodes pendant le même cycle du coeur de sorte qu'il y a un intervalle de temps (dT) entre elles, dans lequel le circuit de commande (34) est agencé de sorte que ledit intervalle de temps (dT) est variable,
**caractérisé en ce que** le circuit de commande (34) comprend des moyens (23) adaptés pour analyser au moins un paramètre dans ledit ECG en champ lointain, ledit paramètre étant une mesure de la synchronisation entre lesdits premier et second ventricules,
ledit circuit de commande (34) comprenant des moyens (35) pour varier l'intervalle de temps (dT) entre les impulsions de stimulation en réaction à l'analyse d'ECG en champ lointain effectuée par lesdits moyens d'analyse (23) jusqu'à ce que ladite synchronisation entre lesdits premier et second ventricules soit optimisée.

2. Appareil implantable pour la stimulation cardiaque selon la revendication 1, **caractérisé en ce que** ledit paramètre dans l'ECG en champ lointain est la durée de QRS.

3. Appareil implantable pour la stimulation cardiaque selon la revendication 1, **caractérisé en ce que** ledit paramètre dans l'ECG en champ lointain est la morphologie de QRS.

4. Appareil implantable pour la stimulation cardiaque selon les revendications 1 à 3, **caractérisé en ce qu'**au moins une électrode (6) non endocardique courte est utilisée pour capter ledit ECG en champ lointain.

5. Appareil implantable pour la stimulation cardiaque selon la revendication 1, **caractérisé en ce qu'**au moins un point d'électrode (7) est situé sur l'encapsulation du stimulateur cardiaque implantable pour capter ledit ECG en champ lointain.

6. Appareil implantable pour la stimulation cardiaque selon les revendications 1 à 3, **caractérisé en ce qu'**au moins une électrode annulaire (9, 10) située sur une électrode endocardique est utilisée pour capter ledit ECG en champ lointain.
